# EUROPÄISCHE PATENTANMELDUNG

(11) **EP 2 711 717 A2**
(43) Veröffentlichungstag der Anmeldung: **26.03.2014**
(21) Anmeldenummer: 13185600.7
(22) Anmeldetag: 23.09.2013
(51) Int. Cl.: G01N 35/10, G01N 21/05

(54) **Detektionsvorrichtung und Detektionsverfahren zur automatischen Bestimmung von Biomasse**

(30) Priorität: 25.09.2012 DE 102012109026
(71) Anmelder: EADS Deutschland GmbH, 85521 Ottobrunn (DE)
(72) Erfinder: Heller, Christoph, 82024 Taufkirchen (DE)
(74) Vertreter: Flügel Preissner Kastel Schober

(57) **Zusammenfassung**

Die Erfindung betrifft eine Detektionsvorrichtung (10) und ein Verfahren zur automatischen Bestimmung von Biomasse (12) in einer Probe (14). Die Detektionsvorrichtung (10) weist eine Fluidikeinrichtung (16) zum automatischen Leiten der Probe (14) durch die Detektionsvorrichtung (10) auf, wobei die Fluidikeinrichtung (16) zum automatischen Aktivieren von Chemolumineszenz in der Probe (14) ausgebildet ist und eine Erfassungseinrichtung (54) zum Erfassen von von der Probe (14) emittierten Chemolumineszenzphotonen (60) aufweist.

## Beschreibung

Die Erfindung betrifft eine Detektionsvorrichtung sowie ein Detektionsverfahren zur automatischen Bestimmung von Biomasse.

Nachweise von beispielsweise Bakterien, Algen oder anderen biologischen Spezies wie z.B. Keimen in Flüssigkeiten werden routinemäßig in der Qualitätskontrolle von insbesondere Trinkwasser, Grundwasser oder auch Badegewässern eingesetzt. Die Menge der in den untersuchten Flüssigkeiten vorhandenen biologischen Spezies gibt einen Hinweis auf ihre hygienische Qualität. Durch die flächendeckende Einführung solcher routinemäßiger Qualitätskontrollen kann die Ausbreitung insbesondere von Seuchen über das Wasser wirkungsvoll verhindert werden.

Übliche Verfahren zum Nachweis von biologischen Spezies sind beispielsweise Kultivierungsmethoden, bei denen zum Beispiel Wasserproben mit geeigneten Medien inkubiert werden, um nach Inkubation die Art und Konzentration von Partikeln in der Probe bestimmen zu können.

Solche bekannten Kultivierungsmethoden haben jedoch eine Reihe von Nachteilen, da sie insbesondere eine lange Inkubationszeit von wenigstens 24 Stunden benötigen und viele manuelle Schritte zur Aufbereitung der Proben und Inkubationsmedien nötig sind, die ein erfahrenes Personal erforderlich machen. Zusätzlich sind Unsicherheitsfaktoren durch beispielsweise manuelle Auszählung diesen Methoden immanent. Weiter muss eine entsprechende Infrastruktur zur Durchführung der bekannten Methoden, beispielsweise in Form von aufwändigen Laboren, bereitgestellt werden.

Daher ist es gewünscht, alternative Vorrichtungen und Verfahren bereitzustellen, mit denen schnellere und sicherere Kontrollen der hygienischen Qualität von Flüssigkeiten möglich sind. Insbesondere sind Vorrichtungen und Verfahren gewünscht, die die Kontrollen automatisch, d.h. weitgehend ohne Einsatz von geschultem Personal, durchführen können.

Beispiele für solche Verfahren als Alternative zu den bekannten Kultivierungsmethoden sind in DE 10 2007 021 387 A1, in DE 10 2008 035 771 B4, DE 10 2008 035 770 A1, DE 10 2009 048 811 A1 und DE 10 2009 048 790 A1 offenbart. Bei den genannten Beispielen werden die biologischen Spezies in automatischen Verfahren auf Filtermaterialien aufkonzentriert und über automatisierte mikroskopische Vorrichtungen auf dem Filtermaterial erfasst.

Der Erfindung liegt die Aufgabe zu Grunde, eine Alternative zu den genannten Beispielen vorzuschlagen, mit der die hygienische Qualität einer Flüssigkeit automatisch kontrolliert werden kann.

Diese Aufgabe wird mit einer Detektionsvorrichtung mit der Merkmalskombination des Anspruchs 1 gelöst.

Ein Verfahren zur automatischen Bestimmung von Biomasse in einer Probe ist Gegenstand des Nebenanspruches.

Vorteilhafte Ausgestaltungen der Erfindung sind Gegenstand der Unteransprüche.

Eine Detektionsvorrichtung zur automatischen Bestimmung von Biomasse in einer Probe weist eine Fluidikeinrichtung zum automatischen Leiten der Probe durch die Detektionsvorrichtung auf. Die Fluidikeinrichtung ist zum automatischen Aktivieren von Chemolumineszenz in der Probe ausgebildet und weist eine Erfassungseinrichtung zum Erfassen von von der Probe emittierten Chemolumineszenzphotonen auf.

Biomasse ist ein so genannter "Summenparameter", über den weitgehend alle in einer Flüssigkeit befindlichen biologischen Spezies erfasst werden können. Dieser Summenparameter ist ein Maß für die hygienische Qualität der Flüssigkeit, denn obwohl Flüssigkeiten, die biologische Spezies beinhalten, nicht unbedingt gesundheitsgefährdend sein müssen, kann eine zu hohe Biomasse, insbesondere einer Veränderung der normalen Menge der Biomasse in einer bekannten Flüssigkeit, ein ernst zu nehmender Hinweis auf unerwünschtes mikrobielles Wachstum in der Flüssigkeit sein. Daher kann die Erfassung der Biomasse in einer Flüssigkeit als schneller Hinweis für weitere, genauere Untersuchungen dienen, die sich erübrigen, wenn der Summenparameter Biomasse keinen Anlass zur Sorge gibt.

Chemolumineszenz entsteht durch Reaktion zweier oder mehrerer Substanzen, wobei durch die Reaktion Energie in Form von Licht Photonen ausgesendet wird. Chemolumineszenz benötigt im Gegensatz zu anderen Lumineszenzarten keine externe energetische Anregung. Es kann daher im Vergleich zu anderen optischen Nachweismethoden auf Anregungsquellen wie beispielsweise Laser verzichtet werden.

Allerdings wurden bislang die Proben, die zur Chemolumineszenzreaktion gebracht werden sollen, zuvor manuell aufbereitet und auch manuell zu einer Erfassungseinrichtung geführt.

Daher wird nun eine automatische Fluidikeinrichtung bereitgestellt, die automatisch eine Probe durch die Detektionsvorrichtung leitet. Gleichzeitig ermöglicht die Fluidikeinrichtung auch eine automatische Aktivierung der Chemolumineszenz der in der Probe vorhandenen Biomasse, so dass die ausgestrahlten Chemolumineszenzphotonen von der Erfassungsseinrichtung detektiert werden können. Somit ist es nun möglich, die Erfassung der in der Probe vorhandenen Biomasse automatisch anhand von Chemolumineszenz durchzuführen.

Es muss somit kein geschultes Personal vorgesehen werden, das manuell die Probe aufbereitet und zu der Erfassungseinrichtung führt, da beide Aufgaben durch die Fluidikeinrichtung übernommen werden.

Vorzugsweise weist die Fluidikeinrichtung eine Mischeinrichtung zum automatischen Mischen der Probe mit wenigstens einem Chemolumineszenzaktivator auf.

In der Mischeinrichtung kann dann vorteilhaft die Probe bzw. die darin enthaltene Biomasse mit dem Chemolumineszenzaktivator zur Reaktion gebracht werden, um die Biomasse vorzugsweise in der Erfassungseinrichtung erfassbar zu machen.

Die Mischeinrichtung ist besonders bevorzugt als Pumpeinrichtung zum Pumpen der Probe und/oder des Chemolumineszenzaktivators und/oder einer Mischung aus Probe und Chemolumineszenzaktivator durch die Detektionsvorrichtung ausgebildet.

Somit kann zum Leiten der Probe von und/oder zu Elementen der Detektionvorrichtung vorteilhaft auf zusätzliche Pumpeinrichtungen verzichtet werden, was den Aufbau der Detektionvorrichtung vorzugsweise vereinfacht.

In bevorzugter Ausgestaltung weist die Mischeinrichtung eine Kolbenspritze mit Kolben und Spritzenzylinder auf.

Kolbenspritzen sind besonders einfach betätigbar und kostengünstig erhältlich und können vorteilhaft sehr einfach bezüglich des zu handhabbaren Volumens gesteuert werden. Zusätzlich ist vorteilhaft eine Mischung von in dem Spritzenzylinder vorhandenen Flüssigkeiten durch Auf- und Abbewegen des Kolbens der Kolbenspritze möglich, so dass vorteilhaft auf weitere Einrichtungen, die eine Mischung von Flüssigkeiten herbeiführen, verzichtet werden kann.

Bevorzugt ist die Erfassungseinrichtung an dem vorzugsweise lichtdurchlässigen Spritzenzylinder angeordnet. Alternativ kann hierfür auch eine separate Kammer, z.B. in Form einer Küvette vorgesehen werden, an welcher die Erfassungseinrichtung angebracht ist und in welche das Proben-Chemolumineszenzaktivator-Gemisch mittels der Spritzenpumpe eingebracht wird.

So können Chemolumineszenzphotonen, die bei Reaktionen der Biomasse in der Probe mit einem Chemolumineszenzaktivator ausgestrahlt werden, vorzugsweise unmittelbar von der Erfassungseinrichtung erfasst werden. Dies führt vorteilhaft zu einer hohen Messgenauigkeit.

Vorteilhaft weist die Mischeinrichtung eine Heizeinrichtung zum Heizen der Probe und/oder des Chemolumineszenzaktivators und/oder einer Mischung aus Probe und Chemolumineszenzaktivator auf.

Damit kann vorteilhaft die Reaktion von Biomasse mit dem Chemolumineszenzaktivator gefördert oder sogar erst ermöglicht werden.

Besonders bevorzugt weist die Fluidikeinrichtung wenigstens ein Reservoir zur Lagerung eines Chemolumineszenzaktivators und/oder von Reinigungsreagenzien zur Reinigung der Fluidikeinrichtung und/oder ein Abfallgefäß zur Aufnahme von Abfallflüssigkeiten auf.

So ist es vorzugsweise möglich, der Probe automatisch das entsprechende Reagenz zum Initiieren der Chemolumineszenz zuzuführen bzw. eine abreagierte Probe vorteilhaft aus beispielsweise dem Bereich der Erfassungseinrichtung zu entfernen. Weiter ist es vorteilhaft möglich, die einzelnen Elemente der Fluidikeinrichtung automatisch zu reinigen.

Vorzugsweise weist das wenigstens eine Reservoir einen Chemolumineszenzaktivator zum Aktivieren von Chemolumineszenz durch Reaktion mit ATP, insbesondere eine Mischung aus Luciferase, D-Luciferin und Mg²⁺, auf.

Eine verbreitete und daher gut untersuchte vorteilhafte Methode zur Bestimmung von Biomasse, insbesondere von lebender Biomasse, ist die Messung des ATP - Gehaltes in Zellen. ATP kann Energie in biochemisch gebundener Form transportieren und ist damit ein Schlüsselmolekül in biologischen Spezies. In toten Zellen wird es sehr schnell abgebaut, dadurch eignet sich ATP vorzugsweise als Indikator für lebende Zellen. Die Menge an ATP in einer Probe spiegelt vorteilhaft die Menge an Biomasse darin wieder.

ATP reagiert beispielsweise mit D - Luciferin und Sauerstoff unter Aussendung von Lichtphotonen, die proportional sind zur vorhandenen Menge des ATP. Die Reaktion wird durch das Enzym Luciferase in Anwesenheit von Mg²⁺ katalysiert.

Alternativ oder zusätzlich weist das wenigstens eine Reservoir einen Chemolumineszenzaktivator zum Aktivieren von Chemolumineszenz durch Reaktion mit Enzymen, insbesondere mit alkalischer Phosphatase, auf. In bevorzugter Ausgestaltung weist dieser Chemolumineszenzaktivator wenigstens ein Dioxetan-Derivat auf.

Ein weit verbreitetes Enzym in lebenden Zellen ist alkalische Phosphatase, deren Nachweis sowohl über farbstoffbildende oder fluoreszierende Substrate als auch über Chemolumineszenzsubstrate durchgeführt werden kann. Besonders bevorzugte Substrate hierfür sind Dioxetan-Derivate.

Alternativ oder zusätzlich weist das wenigstens eine Reservoir einen Chemolumineszenzaktivator zum Aktivieren von Chemolumineszenz durch Reaktion mit Enzymen, insbesondere aus folgender Auswahl auf:
ß-Galaktosidase,
ß-Glucuronidase,
ß-D-Glucosidase,
L - alanine - aminopeptidase;
Peroxidase;
Arylesterase;
Arysulfatase;
Neuraminidase.

Vorteilhaft weist die Fluidikeinrichtung wenigstens eine Filtereinrichtung zum Filtern der Probe auf, die insbesondere einen Porenfilter und/oder ein Filtratreservoir aufweist.

So ist es vorzugsweise möglich, auch eine spezielle interessierende Biomasse zu erfassen, indem andere, nicht interessierende Biomassespezies vorteilhaft aus der Probe herausgefiltert werden können. In diesem Fall wird dann vorteilhaft ein in dem Filtratreservoir aufgefangenes Filtrat für die Erfassung verwendet.

In weiterer bevorzugter Ausgestaltung weist die Fluidikeinrichtung eine Probenaufnahmeeinrichtung zum automatischen Aufnehmen der Probe aus einer Leitung und/oder einem Probengefäß auf.

Somit ist es vorteilhaft auch möglich, automatisch eine Probe aufzunehmen und in die Mischeinrichtung zu überführen, so dass vorzugsweise kein Personal zur Probennahme benötigt wird.

In bevorzugter Ausgestaltung weist die Fluidikeinrichtung eine Lyseeinrichtung zum Lysieren von Zellen in der Probe auf, wobei die Lyseeinrichtung zum chemischen Lysieren und/oder zum mechanischen Lysieren und/oder zum Lysieren der Zellen durch Temperatureinfluss ausgebildet ist.

Insbesondere beim Nachweis von ATP besteht die Problematik, dass das die Reaktion katalysierend Enzym Luciferase nicht in intakte Zellen eindringen kann. Daher ist es bevorzugt, wenn die Zellen der Biomasse vor der Reaktion mit dem Chemolumineszenzaktivator lysiert, d.h. aufgebrochen, werden.

Es ist möglich, die Zellen chemisch, d.h. durch beispielsweise Einfluss von Basen, zu lysieren, alternativ kann eine Lyse aber auch durch mechanische Einflüsse wie beispielsweise Ultraschall oder Scherkräfte erfolgen. Eine weitere alternative Möglichkeit, Zellen zu lysieren besteht darin, sie wiederholt einzufrieren und aufzutauen oder zu erhitzen.

In besonders bevorzugter Ausgestaltung weist die Fluidikeinrichtung mehrere Filtereinrichtungen zum Filtern der Probe auf, wobei eine erste Filtereinrichtung zum Ausfiltern einer interessierenden Biomassespezies aus der Probe und/oder eine zweite Filtereinrichtung zum Ausfiltern von Partikeln größer als die interessierende Biomassespezies, insbesondere von Eukaryoten, aus der Probe ausgebildet ist.

Somit kann beispielsweise eine Klassifizierung der erfassten Biomasse nach Zellgröße durchgeführt werden, da die interessierende Biomassespezies vorzugsweise von anderen, nicht interessierenden, Biomassespezies in der Probe getrennt wird.

Vorteilhaft weist die Fluidikeinrichtung ein Mehrwegeventil zum automatischen Verbinden der Mischeinrichtung mit der Probenaufnahmeeinrichtung und/oder mit der wenigstens einen Filtereinrichtung und/oder mit dem Reservoir für den Chemolumineszenzaktivator und/oder mit dem Reservoir für die Reinigungsreagenzien und/oder mit dem Abfallgefäß auf.

Durch ein solches Mehrwegeventil ist es daher vorteilhaft möglich, die Mischeinrichtung beispielsweise zunächst mit der Probenaufnahmeeinrichtung und danach mit dem Reservoir, in dem der Chemolumineszenzaktivator gelagert ist, zu verbinden. Weiter kann die Mischeinrichtung auch vorteilhaft mit dem Abfallgefäß verbunden werden, um abreagierte Probenflüssigkeit vorzugsweise aus der Mischeinrichtung zu entfernen, so dass vorteilhaft eine frische Probe in derselben Mischeinrichtung untersucht werden kann.

Vorteilhaft weist die Erfassungseinrichtung einen Photodetektor, insbesondere einen Photomultiplier und/oder eine Photodiode auf.

In besonders bevorzugter Ausgestaltung ist eine Steuereinrichtung zum automatischen Ansteuern der Elemente der Fluidikeinrichtung vorgesehen.

Somit können die Elemente der Fluidikeinrichtung vorteilhaft automatisch bedient werden, ohne geschultes Personal zu benötigen, so dass beispielsweise eine vorteilhaft in regelmäßigen Abständen automatisch gesteuerte Überwachung von beispielsweise einer Wasserleitung realisiert werden kann.

Vorzugsweise ist eine Auswerteeinrichtung zum Auswerten der von der Erfassungseinrichtung erfassten Chemolumineszenzphotonen vorgesehen.

Eine solche Auswerteeinrichtung kann beispielsweise nicht nur aus den erfassten Chemolumineszenzphotonen die Menge an Biomasse in einer Probe bestimmen, sondern beispielsweise auch Alarmsignale ausgeben, wenn die Menge der Biomasse einen vorbestimmten Grenzwert übersteigt.

In einer möglichen Ausgestaltung weist die Auswerteeinrichtung eine Vergleichseinheit zum Vergleichen der Menge der erfassten Chemolumineszenzphotonen mit zuvor bestimmten Datensätzen auf, in denen die Menge der Chemolumineszenzphotonen und die Menge an Biomasse in Abhängigkeit zueinander gesetzt sind. Dazu ist in der Auswerteeinrichtung insbesondere eine Speichereinheit zum Speichern von bekannten Datensätzen vorgesehen.

Ein Verfahren zur automatischen Bestimmung von Biomasse in einer Probe weist die folgenden Schritte auf:
a) automatische Aufnahme der Probe mit der Biomasse in eine Fluidikeinrichtung;
b) automatisches Aktivieren von Chemolumineszenz der Probe in der Fluidikeinrichtung;
c) Erfassen von von der Probe emittierten Chemolumineszenzphotonen.

Vorteilhaft wird die Probe vor Schritt b) automatisch wenigstens einmal gefiltert, wobei zum Bilden wenigstens eines Filtrats der Probe insbesondere größere Partikel als eine interessierende Biomassespezies und/oder die interessierende Biomassespezies aus der Probe gefiltert werden.

Vorzugsweise wird in Schritt b) die Probe automatisch mit einem Chemolumineszenzaktivator zur Reaktion gebracht, insbesondere mit einem Chemolumineszenzaktivator, der mit ATP und/oder einem Enzym, insbesondere mit alkalischer Phosphatase, reagiert.

Besonders bevorzugt werden Zellen in der Probe in Schritt b) automatisch lysiert.

Vorteilhaft werden in Schritt c) Chemolumineszenzphotonen in einem ersten Filtrat aus der Probe ohne darin vorhandener interessierender Biomasse und in einem zweiten Filtrat aus der Probe mit darin vorhandener interessierender Biomasse erfasst, wobei eine Differenz der erfassten Chemolumineszenzphotonen gebildet wird.

Dadurch ist es vorteilhaft möglich, Chemolumineszenzphotonen, die von Biomassespezies stammen, die nicht von Interesse sind, aus denen erfassten Chemolumineszenzphotonen herauszurechnen. Es ergibt sich somit vorzugsweise ein Wert, der vorteilhaft nur von der interessierenden Biomasse stammt.

Vorzugsweise wird nach Schritt c) der Schritt Auswerten der erfassten Chemolumineszenzphotonen durch Vergleich mit zuvor erfassten Datensätzen durchgeführt.

Bevorzugt werden Schritte des Verfahrens in einer Detektionvorrichtung durchgeführt, wobei die Elemente der Detektionsvorrichtung derart angesteuert werden, dass die Schritte automatisch erfolgen.

In bevorzugter Ausgestaltung wird vor Schritt a) wenigstens teilweise eine Reinigung der Elemente der Detektionsvorrichtung durchgeführt.

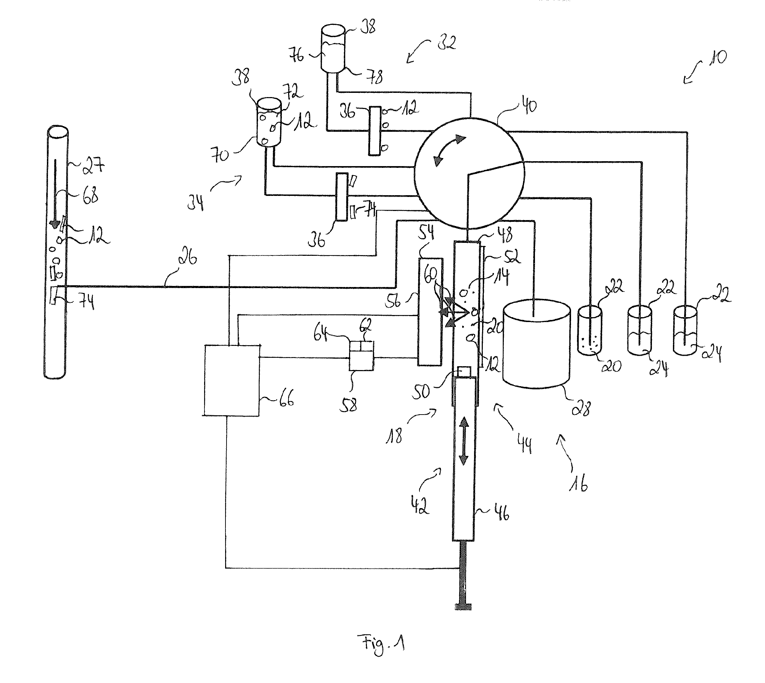

Im Folgenden wird die Erfindung anhand der beigefügten Zeichnung Fig. 1 näher erläutert.

Dabei zeigt Fig. 1 eine Detektionvorrichtung 10 zur Bestimmung von Biomasse 12 in einer Probe 14.

Die Detektionvorrichtung 10 weist eine Fluidikeinrichtung 16 auf.

Die Fluidikeinrichtung 16 umfasst eine Mischeinrichtung 18, in der die Biomasse 12 automatisch mit einem Chemolumineszenzaktivator 20 zur Reaktion gebracht werden kann.

Die Fluidikeinrichtung 16 weist weiter mehrere Reservoire 22 zur Aufnahme und Lagerung von Reagenzien wie dem Chemolumineszenzaktivator 20 bzw. von Reinigungsreagenzien 24 auf. Zusätzlich sind eine Probenaufnahmeeinrichtung 26 zum automatischen Aufnehmen von Proben 14 aus einer Leitung 27 und ein Abfallgefäß 28 zur Aufnahme von Abfallflüssigkeiten wie beispielsweise einer Probe 14 mit abreagierter Biomasse 12 vorgesehen.

Ferner weist die Fluidikeinrichtung 16 eine erste Filtereinrichtung 32 und eine zweire Filtereinrichtung 34 auf, in denen jeweils ein Porenfilter 36 sowie ein Filtratreservoir 38 angeordnet sind.

Die Mischeinrichtung 18 ist über ein Mehrwegeventil 40 sowohl mit der Probenaufnahmeeinrichtung 26, jedem der Reservoire 22, den Filtereinrichtungen 32, 34, als auch mit dem Abfallgefäß 28 verbindbar.

Die Mischeinrichtung 18 ist in der vorliegenden Ausführungsform als Pumpeinrichtung 42 in Form einer Kolbenspritze 44 ausgebildet.

Die Kolbenspritze 44 weist einen Kolben 46 und einen lichtdurchlässigen Spritzenzylinder 48 auf, in dem der Kolben 46 auf - und abbewegbar ist.

An dem Kolben 46 ist eine Lyseeinrichtung 50 angeordnet, mit Hilfe der Zellen in einer in dem Spritzenzylinder 48 befindlichen Probe 14 lysiert werden können.

Der Spritzenzylinder 48 selbst ist mit einer Heizeinrichtung 52 ausgestattet, mittels der es möglich ist, die Probe 14 in dem Spritzenzylinder 48 zu heizen.

Eine Erfassungseinrichtung 54 in Form eines Photodetektors 56 ist ebenfalls am lichtdurchlässigen Spritzenzylinder 48 angeordnet und weiter mit einer Auswerteeinrichtung 58 verbunden, die von der Probe 14 ausgesandte und von dem Photodetektor 56 erfasste Chemolumineszenzphotonen 60 auswertet. Dazu weist die Auswerteeinrichtung 58 eine Vergleichseinrichtung 62 und eine Speichereinrichtung 64 zum Speichern von zuvor bestimmten Datensätzen auf.

Sämtliche Elemente der Detektionvorrichtung 10 werden durch eine Steuereinrichtung 66 automatisch angesteuert.

Soll nun beispielsweise die hygienische Qualität von Wasser 68 in einer Leitung 27 kontrolliert werden, wird die Mischeinrichtung 18 durch das Mehrwegeventil 40 zunächst mit der Probenaufnahmeeinrichtung 26 verbunden, über die eine Probe 14 aus der Leitung 27 mit darin vorhandener Biomasse 12 aufgenommen werden kann.

Dazu wird der Kolben 46 zurückgezogen, so dass in der Kolbenspritze 44 ein Unterdruck entsteht und Wasser 68 in die Mischeinrichtung 18 gezogen wird.

Nachfolgend wird die Mischeinrichtung 18 über das Mehrwegeventil 40 mit der zweiten Filtereinrichtung 34 verbunden. Der Kolben 46 wird gedrückt, so dass die aufgenommene Probe 14 durch den Porenfilter 36 in ein Filtratreservoir 70 gedrückt wird. Der Porenfilter 36 der zweiten Filtereinrichtung 34 weist eine Porengröße auf, die dazu geeignet ist, Biomasse, die größer ist als bakterielle Biomasse 12, aus dem entstehenden zweiten Filtrat 72, zu entfernen. Soll beispielsweise bakterielle Biomasse 12 erfasst werden, eignet sich ein Porenfilter 36 mit einer Porengröße von etwa 3-5 µm. So werden euch Eukaryoten 74 aus dem zweiten Filtrat 72 entfernt. Sollen dagegen als Biomasse beispielsweise Algen erfasst werden, ist ein Porenfilter 36 mit einer Porengröße von etwa 20 µm geeignet. Nachfolgend wird die Mischeinrichtung 18 über das Mehrwegeventil 40 direkt mit dem Filtratreservoir 70 verbunden. Ein Teil des zweiten Filtrats 72 wird durch Betätigung des Kolbens 46 wieder in die Kolbenspritze 44 eingesaugt und die Stellung des Mehrwegeventil 40 geändert, so dass nun die Mischeinrichtung 18 mit der ersten Filtereinrichtung 32 verbunden ist. Durch erneute Betätigung des Kolbens 46 wird das zweite Filtrat 72 durch den Porenfilter 36 der ersten Filtereinrichtung 32 zum Bilden eines ersten Filtrats 76 in das erste Filtratreservoir 78 gedrückt. Die Porengröße des Porenfilters 36 ist hier geringer als die in der zweiten Filtereinrichtung 34, um die interessierende Biomasse 12, beispielsweise bakterielle Biomasse 12, aus dem ersten Filtrat 76 zu entfernen. Eine geeignete Porengröße wäre hier im Falle von bakterieller Biomasse 12 etwa 0,1 µm und im Falle von Algen - Biomasse 12 etwa 2 µm.

Nachfolgend wird die Mischeinrichtung 18 über das Mehrwegeventil 40 direkt mit dem Filtratreservoir 78 verbunden. Das erste Filtrat 76 wird nun in die Mischeinrichtung 18 gepumpt und die Stellung des Mehrwegeventils 18 so verändert, dass die Mischeinrichtung 18 nun mit dem Reservoir 22 mit darin gelagertem Chemolumineszenzaktivator 20 verbunden ist. Der Chemolumineszenzaktivator 20 wird ebenfalls in die Mischeinrichtung 18 gepumpt und dort durch Auf - und Abbewegung des Kolbens 46 mit dem ersten Filtrat 76 vermischt. Dabei reagiert die in dem ersten Filtrat 76 noch vorhandene Biomasse 12 mit dem Chemolumineszenzaktivator 20, wobei Chemolumineszenzphotonen 60 freigesetzt werden, die von der Erfassungseinrichtung 54 erfasst werden.

Um die Reaktion zu beschleunigen bzw. überhaupt erst zu ermöglichen, kann die Heizeinrichtung 52 die in dem Spritzenzylinder 48 vorhandene Mischung aufheizen bzw. die Zellen der darin vorhandenen Biomasse 12 können durch die Lyseeinrichtung 50 zwecks Reaktion aufgebrochen werden.

Anschließend wird die Stellung des Mehrwegeventil 40 so verändert, dass die Mischeinrichtung 18 mit dem Abfallgefäß 28 verbunden ist, und das abreagierte erste Filtrat 76 wird in das Abfallgefäß 28 gepumpt.

Danach wird die Mischeinrichtung 18 über das Mehrwegeventil 40 direkt mit dem Filtratreservoir 70 verbunden und der in dem zweiten Filtratreservoir 70 verbliebene Rest des zweiten Filtrats 72 in die Mischeinrichtung 18 aufgenommen.

Eventuell wird zuvor durch Verbindung der Mischeinrichtung 18 mit den Reservoiren 22 mit darin gelagerten Reinigungsreagenzien 24 der Spritzenzylinder 48 gereinigt.

Die bereits im Zusammenhang mit dem ersten Filtrat 76 beschriebene Aktivierung von Chemolumineszenz wird in den zweiten Filtrat 72 analog durchgeführt und die Chemolumineszenzphotonen 60 durch die Erfassungseinrichtung 54 erfasst.

Durch das beschriebene Verfahren wurden demnach zunächst Biomassespezies aus der Probe 14 entfernt, die größer sind als die interessierende Biomassespezies.

In der Probe 14 sind jedoch auch Biomassespezies vorhanden, die kleiner sind als die interessierende Biomassespezies. Diese kleinere Biomassespezies wird durch Messung des ersten Filtrats 76 erfasst.

Wird daher das Ergebnis der Messung des ersten Filtrats 76 von dem Ergebnis der Messung des zweiten Filtrats 72 abgezogen, resultiert die Menge an Chemolumineszenzphotonen 60, die tatsächlich von der interessierenden Biomasse stammt.

Es ist jedoch auch möglich, in dem Verfahren lediglich die Gesamt - Biomasse 12 unabhängig von der jeweiligen Spezies zu erfassen, dabei können dann die Filterschritte in dem Verfahren entfallen.

In der Auswerteeinrichtung 58 wird die Menge der erfassten Chemolumineszenzphotonen 60 mit Datensätzen verglichen, die in der Auswerteeinrichtung 58 gespeichert sind, und die eine Abhängigkeit von der Menge der Chemolumineszenzphotonen 60 von der Menge der Biomasse 12 in einer Probe 14 darstellen.

Durch diesen Vergleich kann somit die Menge der in der untersuchten Probe 14 vorhandenen Biomasse 12, eventuell abhängig von einer bestimmten Biomassespezies, erfasst werden.

Das Verfahren wird vollautomatisch durchgeführt, weil sämtliche Elemente der Detektionvorrichtung 10, nämlich die Probenaufnahmeeinrichtung 26, das Mehrwegeventil 40, der Kolben 46, die Erfassungeinrichtung 54 und die Auswerteeinrichtung 58, automatisch durch die Steuereinrichtung 66 angesteuert werden.

Die Menge der aktiven Biomasse 12 in einer Probe 14 wie z.B. Wasser, Umweltproben, Bakterien-, Algen-, oder Zellkulturen ist ein Summenparameter für die hygienische Qualität der Probe 14, bzw. für den Erfolg eines Kultivierungsprozesses. Die derzeitigen Methoden zur Bestimmung der Biomasse 12 sind meist wenig sensitiv und/oder arbeitsaufwändig.

Denn die Nachweisreaktionen für Biomasse 12 sind bekannt, werden aber bis dato manuell durchgeführt.

Das Problem wird durch einen fluidischen Aufbau gelöst, bestehend aus Ventil(en) und Pumpe(n). Damit werden Wasserprobe, Reinigungs- und Puffersubstanzen sowie geeignete Reagenzien gefördert, gemischt und temperiert. Die aktive Menge einer bestimmten Zellpopulation kann durch eine differentielle Messung bestimmt werden.

Die Detektionsvorrichtung 10 ermöglicht eine automatisierte, quasi-kontinuierliche (z.B. in Zeitintervallen von 10-15 Minuten) Analyse der gesamten Biomasse 12 oder der Biomasse 12 einer Teilpopulation in einer Wasserprobe oder einer anderen wässrigen Flüssigkeit.

Es handelt sich bei dem beschriebenen Verfahren demnach um eine automatische Bestimmung aktiver Biomasse 12, beispielsweise in Wasser 68 oder sonstigen wässrigen Lösungen.

Der Nachweis von Bakterien in Wasser 68 wie Trinkwasser, Badegewässer, Mineralwasser, Prozesswasser, Oberflächenwasser, Grundwasser, Abwasser, Kühlwasser, etc., Getränken, z.B. Milch, oder anderen wässrigen Medien wird routinemäßig mit sog. Kultivierungsverfahren durchgeführt. Dabei wird eine bestimmte Menge der (Wasser-)Probe 14 filtriert und das Filter anschließend auf einer Agar-Platte mit geeignetem Medium inkubiert. Bei hohen Keimkonzentrationen kann eine kleine Menge der Probe 14 auch direkt auf die Agar-Platte gegeben werden. Verschiedene Varianten dieses Verfahrens zum spezifischen und unspezifischen Nachweis von Keimen wurden gegen Ende des 19. Jahrhunderts entwickelt und seither fast unverändert in der Routine eingesetzt. Durch die flächendeckende Einführung solcher Analysen konnte die Ausbreitung von Seuchen über das Wasser 68 vor allem in den Industrieländern wirkungsvoll verhindert werden. Obwohl die Kultivierungsmethode schon recht alt ist, erfüllt sie im Alltag ihren Zweck, was die hohe Qualität unseres Trink- und Badewassers beweist.

Kultivierungsmethoden haben jedoch eine Reihe von Nachteilen, insbesondere:
- Hoher Zeitaufwand: erste Ergebnisse nach 24 - 48 Stunden; Endergebnis nach 72 Stunden oder später;
- Nicht automatisierbar;
- Viele manuelle Schritte (Filtration, Plattierung, Auszählung);
- Erfahrenes Personal erforderlich;
- Infrastruktur (Labor) erforderlich.

In vielen Fällen wäre eine schnelle Aussage über die hygienische Qualität des Wassers 68 oder der Flüssigkeit wünschenswert. Hierzu bietet sich u.a. die sogenannte Biomasse 12 als Messparameter an. Dies ist ein "Summenparameter", d.h. es werden alle in der Flüssigkeit befindlichen Zellen erfasst. Obwohl Trinkwasser (oder eine andere Flüssigkeit), welches prokaryotische oder eukaryotische Zellen beinhaltet, nicht per se gesundheitsgefährdend sein muss, kann eine zu hohe Biomasse 12 durchaus ein ernstzunehmender Hinweis darauf sein, dass ein Problem vorliegt. Insbesondere Veränderungen in der Biomasse 12 einer Flüssigkeit können unerwünschtes mikrobielles Wachstum anzeigen.

Die Erfassung der Biomasse 12 kann also als schneller Hinweis und als "Trigger" für weitere, genauere Untersuchungen dienen.

Situationen oder Anwendungen, bei denen ein solcher schneller Nachweis vorteilhaft wäre, sind beispielsweise:
- Kontrolle von wasserführenden Systemen in Verkehrs- und Transportmitteln mit langer Reisezeit bzw. Autonomiezeit (Schiffe, U-Boote, Flugzeuge, Raumkapseln, Raumstationen);
- Kontinuierliche Überwachung von Trinkwasseraufbereitungssystemen;
- Kontinuierliche Überwachung von Wassererzeugungssystemen (z.B. Brennstoffzelle);
- Erkennung von graduellen, aber raschen Veränderungen (innerhalb 1 Tages) der Wasserqualität, hervorgerufen z.B. durch Ablösung von Biofilmen von Rohrleitungen oder Kontamination des Wassers 68 durch Rohrbrüche;
- Erkennung von sehr schnellen Veränderungen der Wasserqualität (innerhalb 1 Stunde), hervorgerufen z.B. durch Naturkatastrophen (Erdbeben, Starkregen), Störungen und Pannen (Durchbruch von Filtern), Unfälle oder absichtliche Kontamination (Bioterrorismus);
- Lebensmittelkontrollen.

Neben der Vermeidung von (mikro-)biologischem Wachstum gibt es jedoch auch die umgekehrte Situation, also Anwendungen, wo solches Wachstum gezielt gefördert werden soll. Solche Kultivierungen werden in zahlreichen biotechnologischen Prozessen eingesetzt. Diese finden meist in sog. Fermentern oder Bioreaktoren statt. Um diese Prozesse optimal zu steuern, werden mehrere Parameter (z.B. Temperatur, pH-Wert, Sauerstoffzufuhr, Nährstoffgehalt) kontinuierlich überwacht, darunter auch die Menge der entstehenden Biomasse 12.

Typische Beispiele für biotechnologische Prozesse sind:
- Anzucht von Algen zur Produktion von Biokraftstoffen;
- Zellkulturen;
- Produktion von Antikörpern und sog. "Biologics";
- Fermentationsprozesse;
- Abwasserbehandlung;
- Biogas-Erzeugung.

Zur Bestimmung der Biomasse 12 wurden bisher verschiedene Methoden entwickelt, so z.B.:
- Mikroskopische Auszählung;
- Messung der Zelldichte über Lichtstreuung;
- Respirationsverfahren;
- Proteinbestimmung;
- Bestimmung des organischen Kohlenstoffs;
- Impedanzmessung.

Diese Methoden sind meist zeitaufwändig und erfordern viele manuelle Schritte. Sie sind auch nur selten für hochsensitive Bestimmungen geeignet.

Zwei weitere, spezielle Methoden zur Biomassebestimmung sind:

### a) ATP-Gehalt

Eine vor allem in der Mikrobiologie verbreitete Methode zur Bestimmung der aktiven (also "lebenden") Biomasse 12, ist die Messung des ATP (Adeno-sintriphosphat)-Gehalts in der Zelle. ATP ist die energetische "Währung" einer Zelle; es kann Energie in biochemisch gebundener Form transportieren und ist damit ein Schlüsselmolekül für die Zelle. In toten Zellen wird es hingegen sehr schnell abgebaut. Dadurch eignet sich ATP als universeller Indikator für lebende Zellen. Die Menge an ATP in einer Probe 14 spiegelt die Menge an Biomasse 12 darin wider.

Der Nachweis von ATP erfolgt über folgende Reaktion:

ATP + D-Luciferin + O₂ → Oxyluciferin + PPi + AMP + CO₂ + Licht

AMP = Adenosinmonophopshat;
PPi = anorganisches Pyrophosphat

Diese Reaktion wird durch das Enzym Luciferase aus dem Glühwürmchen (Photinus pyralis) in Anwesenheit von Mg²⁺ katalysiert. Die Menge des abgestrahlten Lichts ist dabei proportional zur vorhandenen ATP-Menge.

Da das Enzym Luciferase nicht in intakte Zellen eindringen kann, werden diese vor der Messung bevorzugt lysiert.

### b) Enzym-Nachweis

Die biologische Aktivität einer Probe 14 kann auch mittels eines Enzymnachweises ermittelt werden. Als Parameter wird hier häufig das ubiquitär (weit verbreitet) vorkommende Enzym Alkalische Phosphatase verwendet. Sein Vorkommen ist ein Indikator für das Vorhandensein lebender Zellen. Der Nachweis kann über farbstoffbildende oder fluoreszierende Substrate erfolgen; es wurden jedoch auch chemilumineszente Substrate (Dioxetan- Derivate) dafür synthetisiert, wie beispielsweise ein 1,2-Dioxetan durch enzymatischen Hydrolyse, mit dem folgendermaßen Lumineszenz erzeugt werden kann:

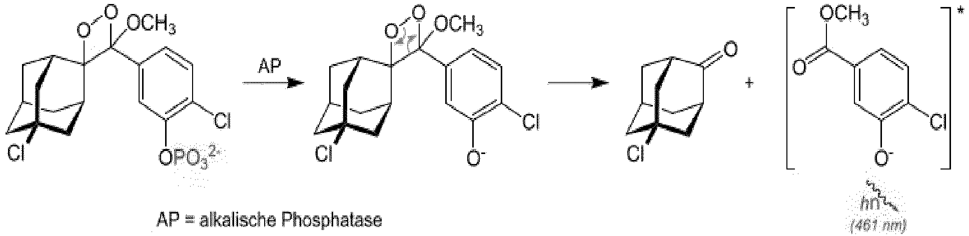

Die Verwendung chemilumineszenter Substrate zum Enzymnachweis hat gegenüber anderen Methoden (Farbsubstrate, Fluoreszenzsubstrate) generell folgende Vorteile:
- Geringer apparativer Aufwand, da zum Nachweis lediglich ein Photodetektor 56 (z.B. Photomuliplier, Photodiode) erforderlich ist. Die Notwendigkeit einer Anregungslichtquelle und von optischen Filtern entfällt;
- Keine Störung (Hintergrund) durch das Licht einer Anregungslichtquelle und somit eine höhere Empfindlichkeit;
- Längere Messzeiten (Signalintegration) sind möglich, da es kein Ausbleichen des Farbstoffs gibt;
- Automatisierung.

Die o.g. beiden chemilumineszenten Nachweisreaktionen für Biomasse 12 werden bis dato manuell durchgeführt. Im Folgenden wird eine Technologie beschrieben, welche mehrere Aufgaben erfüllt:
- Vollautomatische Messung der Biomasse 12 via ATP oder Alkalische Phosphatase;
- Messung "on-line", also direkt an einer Wasserleitung oder einem Flüssigkeitsbehälter;
- Messung in kurzer Zeit (ca. 15 min);
- Klassifizierung der Biomasse 12 nach Zellgröße.

Die zu untersuchende Flüssigkeit wird mittels einer Spritzenpumpe aus einem Behälter oder einer Leitung 27 angesaugt und durch ein grobporiges Filter in das Filtratreservoir 70 gepumpt.

Soll bakterielle Biomasse 12 bestimmt werden, so wird bevorzugt ein Filter mit einer Porengröße von ca. 3-5 µm eingesetzt. Dadurch werden größere, eukaryotische Zellen zurückgehalten, wohingegen die bakteriellen Zellen im Filtrat 72 bleiben.

Zur Bestimmung der Biomasse 12 von Algen (ca. 5-10 µm Durchmesser) wird bevorzugt ein Filter mit entsprechend größeren Poren (z.B. 20 µm) eingesetzt.

Zur Steuerung der Flüssigkeitsströme dient das Mehrwegeventil 40.

Aus dem Filtratreservoir 70 wird mittels Spritzenpumpe etwa die Hälfte der Flüssigkeit entnommen und durch ein feinporiges Filter in das Filtratreservoir 78 gepumpt. Die Porengröße dieses Filters ist bevorzugt geringer als die Größe der zu untersuchenden Organismen. Dies wäre z.B. etwa 0,1 µm Porengröße für Bakterien-Biomasse 12 und etwa 2 µm Porengröße für Algen-Biomasse 12.

Nach dieser Größenfraktionierung erfolgen die Messungen.

Dazu wird zunächst eine definierte Menge Filtrat 76 mittels Spritzenpumpe aus Reservoir 78 entnommen. Anschließend wird eine definierte Menge Detektionsreagenz aufgenommen und beide Flüssigkeiten durch Auf- und Abbewegen des Spritzenkolbens vermischt. Abhängig von der Art der Biomassenbestimmung befindet sich im Gefäß entweder ein chemilumineszentes Substrat für Alkalische Phosphatase oder ein Reaktionsmix (Enzym, Substrat) für den ATP-Nachweis.

Nahe am Spritzenzylinder 48 aus Glas befindet sich ein Photodetektor 56, z.B. ein Photomultiplier, welcher das von der Reaktion ausgesandte Licht detektiert. Das Signal wird verstärkt und von einem geeigneten System (z.B. Computer) aufgezeichnet.

Mittels einer zuvor erstellten Kalibrierkurve (Lichtausbeute vs. ATP-Menge bzw. Alkalische Phosphatase-Menge) kann auf die in der Probe 14 vorhandene ATP-Menge (bzw. Phosphatase-Menge) und damit auf die Biomasse 12 zurückgeschlossen werden.

Die in der ersten Messung bestimmte Größe ist also ein Maß für das sich in freier Lösung befindliche ATP bzw. die sich in freier Lösung befindliche Alkalische Phosphatase. Im Falle der Biomassebestimmung von Algen befindet sich in dieser Fraktion zusätzlich auch bakterielle Biomasse 12.

Nach der Messung wird die Spritze in den Abfall entleert und mit einer definierten Menge Filtrat 72 befüllt. Anschließend wird erneut eine definierte Menge Detektionsreagenz aufgenommen und beide Flüssigkeiten durch Auf- und Abbewegen des Spritzenkolbens vermischt. Das nach Vermischen entstehende Licht wird erneut vom Detektor registriert.

Die in der zweiten Messung bestimmte Größe ist ein Maß für die Summe aus freiem ATP (bzw. freier Alkalischer Phosphatase) und dem bakteriellen ATP (bzw. bakterieller Alkalischer Phosphatase).

Für den Fall der Biomassebestimmung aus Algen gilt: Filtrat 76 = freies ATP + bakterielles ATP +zelluläres ATP (bzw. Alkalische Phosphatase).

Durch Subtraktion der beiden Werte (Messung 2 - Messung 1) erhält man die Netto-Biomasse 12, also die Biomasse 12 der Bakterien (bzw. der Algen).

Nach Abschluß der Messungen werden die Pumpe, das Ventil, die Filtratreservoire 70, 78 sowie die Schläuche automatisch geleert und mit Reinigungsflüssigkeiten gereinigt.

Abhängig von der Filterkapazität (Filterfläche) und der Zell- bzw. Partikelzahl in der Flüssigkeit können die Filter mehrfach verwendet werden. Vorteilhaft ist dabei die Verwendung von mikromechanischen Filtern, die bei entsprechender Reinigung bis zu 100 mal wiederverwendet werden können.

Der gesamte Filtrations-, Mess- und Reinigungsvorgang wird über ein Computerprogramm gesteuert.

Entsprechend der Verfügbarkeit geeigneter chemilumineszenter Substrate können mit dieser Technologie auch andere Enzyme als sog. "Biomarker" vollautomatisch nachgewiesen werden. Beispielsweise wären dies:
- AMPGD (3-(4-methoxyspiro [1,2-dioxetane-3,2'-tricyclo-[3.3.1. 1(3,7)]decan]-4- yl) phenyl-beta-0-galactopyranoside) zum Nachweis des Enzyms β-Galaktosidase. Dieses Enzym ist generell ein Indikator für coliforme Keime.
- Glucuronide-substituted dioxetane; zum Nachweis des Enzyms ß-Glucuronidase. Dieses Enzym ist ein Indikator für E. coli.
- Luminol, zusammen mit Wasserstoff-Peroxid; zum Nachweis des Enzyms Peroxidase.
- Arylesterase chemiluminescent substrate, zum Nachweis von Arylesterase. Arylesterase ist ein wichtiger Biomarker im Serum und steht in Verbindung mit verschiedenen Stoffwechsel- und Kreislauferkrankungen.
- Arylsulfatase chemiluminescent substrate, zum Nachweis von Arylsulfatase. Ein Mangel an diesem Enzym führt beim Menschen zu einer Stoffwechselstörung.
- Neuraminidase chemiluminescent substrate, zum Nachweis der Neuraminidase. Dieses Enzym kommt beispielsweise in Viren (Masern, Mumps, Influenza) vor und dient zur Abspaltung des Viruspartikels von der Wirtszelle. Über den Nachweis dieses Enzyms lassen sich die entsprechen den Viren in einer Blut- oder Serumprobe nachweisen.
- Alkalische Phosphatase, ß-Glucuronidase und ß-Galaktosidase werden auch in sog. "reporter gene assays" eingesetzt. Dabei wird in die DNA eines Organismus das Gen für eines der genannten Enzyme hinter das zu unter suchende Gen oder hinter den zu untersuchenden Promotor eingesetzt. Die Menge an erzeugtem chemilumineszenten Licht erlaubt eine Aussage über die entsprechende Gen-Aktiviät bzw. Promotor-Aktivität. Mit oben beschriebenem System können also auch Reporter Gene Assays vollautomatisch durchgeführt werden.

### Bezugszeichenliste

- 10: Detektionsvorrichtung
- 12: Biomasse
- 14: Probe
- 16: Fluidikeinrichtung
- 18: Mischeinrichtung
- 20: Chemolumineszenzaktivator
- 22: Reservoir
- 24: Reinigungsreagenz
- 26: Probenaufnahmeeinrichtung
- 27: Leitung
- 28: Abfallgefäß
- 32: erste Filtereinrichtung
- 34: zweite Filtereinrichtung
- 36: Porenfilter
- 38: Filtratreservoir
- 40: Mehrwegeventil
- 42: Pumpeinrichtung
- 44: Kolbenspritze
- 46: Kolben
- 48: Spritzenzylinder
- 50: Lyseeinrichtung
- 52: Heizeinrichtung
- 54: Erfassungseinrichtung
- 56: Photodetektor
- 58: Auswerteeinrichtung
- 60: Chemolumineszenzphoton
- 62: Vergleichseinrichtung
- 64: Speichereinrichtung
- 66: Steuereinrichtung
- 68: Wasser
- 70: zweites Filtratreservoir
- 72: zweites Filtrat
- 74: Eukaryot
- 76: erstes Filtrat
- 78: erstes Filtratreservoir

## Patentansprüche

1. Detektionsvorrichtung (10) zur automatischen Bestimmung von Biomasse (12) in einer Probe (14) mit einer Fluidikeinrichtung (16) zum automatischen Leiten der Probe (14) durch die Detektionsvorrichtung (10),
wobei die Fluidikeinrichtung (16) zum automatischen Aktivieren von Chemolumineszenz in der Probe (14) ausgebildet ist und eine Erfassungseinrichtung (54) zum Erfassen von von der Probe (14) emittierten Chemolumineszenzphotonen (60) aufweist.

2. Detektionsvorrichtung (10) nach Anspruch 1,
**dadurch gekennzeichnet, dass** die Fluidikeinrichtung (16) eine Mischeinrichtung (18) zum automatischen Mischen der Probe (14) mit wenigstens einem Chemolumineszenzaktivator (20) aufweist, die insbesondere als Pumpeinrichtung (42) zum Pumpen der Probe (14) und/oder des Chemolumineszenzaktivators (20) und/oder einer Mischung aus Probe (14) und Chemolumineszenzaktivator (20) durch die Detektionsvorrichtung (10) ausgebildet ist.

3. Detektionsvorrichtung (10) nach Anspruch 2,
**dadurch gekennzeichnet, dass** die Mischeinrichtung (18) eine Kolbenspritze (44) mit Kolben (46) und Spritzenzylinder (48) aufweist, wobei die Erfassungseinrichtung (54) insbesondere an dem Spritzenzylinder (48) angeordnet ist und/oder dass die Mischeinrichtung (18) eine Heizeinrichtung (52) zum Heizen der Probe (14) und/oder des Chemolumineszenzaktivators (20) und/oder einer Mischung aus Probe (14) und Chemolumineszenzaktivator (20) aufweist.

4. Detektionsvorrichtung (10) nach einem der voranstehenden Ansprüche,
**dadurch gekennzeichnet, dass** die Fluidikeinrichtung (16) wenigstens ein Reservoir (22) zur Lagerung eines Chemolumineszenzaktivators (20) und/oder von Reinigungsreagenzien (24) zur Reinigung der Fluidikeinrichtung (16) und/oder ein Abfallgefäß (28) zur Aufnahme von Abfallflüssigkeiten aufweist.

5. Detektionsvorrichtung (10) nach Anspruch 4,
**dadurch gekennzeichnet, dass** das wenigstens eine Reservoir (22) einen Chemolumineszenzaktivator (20) zum Aktivieren von Chemolumineszenz durch Reaktion mit ATP, insbesondere eine Mischung aus Luciferase, D-Luciferin und Mg²⁺, und/oder zum Aktivieren von Chemolumineszenz durch Reaktion mit Enzymen, insbesondere mit alkalischer Phosphatase, aufweist, wobei der Chemolumineszenzaktivator (20) insbesondere wenigstens ein Dioxetan-Derivat aufweist.

6. Detektionsvorrichtung (10) nach einem der voranstehenden Ansprüche,
**dadurch gekennzeichnet, dass** die Fluidikeinrichtung (16) wenigstens eine Filtereinrichtung (32, 34) zum Filtern der Probe (14) aufweist, die insbesondere einen Porenfilter (36) und/oder ein Filtratreservoir (70, 78) aufweist und/oder dass die Fluidikeinrichtung (16) eine Probenaufnahmeeinrichtung (26) zum automatischen Aufnehmen der Probe (14) aus einer Leitung (27) und/oder einem Probengefäß aufweist und/oder dass die Fluidikeinrichtung (16) eine Lyseeinrichtung (50) zum Lysieren von Zellen in der Probe (14) aufweist, wobei die Lyseeinrichtung (50) zum chemischen Lysieren und/oder zum mechanischen Lysieren und/oder zum Lysieren der Zellen durch Temperatureinfluss ausgebildet ist.

7. Detektionsvorrichtung (10) nach einem der voranstehenden Ansprüche,
**dadurch gekennzeichnet, dass** die Fluidikeinrichtung (16) mehrere Filtereinrichtungen (32, 34) zum Filtern der Probe (14) aufweist, wobei eine erste Filtereinrichtung (32) zum Ausfiltern einer interessierenden Biomassespezies aus der Probe (14) und/oder eine zweite Filtereinrichtung (34) zum Ausfiltern von Partikeln größer als die interessierende Biomassespezies, insbesondere von Eukaryoten (74), aus der Probe (14) ausgebildet ist.

8. Detektionsvorrichtung (10) nach Anspruch 6 oder nach Anspruch 4,
**dadurch gekennzeichnet, dass** die Fluidikeinrichtung (16) ein Mehrwegeventil (40) zum automatischen Verbinden der Mischeinrichtung (18) mit der Probenaufnahmeeinrichtung (26) und/oder mit der wenigstens einen Filtereinrichtung (32, 34) und/oder mit dem Reservoir (22) für den Chemolumineszenzaktivator (20) und/oder mit dem Reservoir (22) für die Reinigungsreagenzien (24) und/oder mit dem Abfallgefäß (28) aufweist.

9. Detektionsvorrichtung (10) nach einem der voranstehenden Ansprüche,
**dadurch gekennzeichnet, dass** die Erfassungseinrichtung (54) einen Photodetektor (56), insbesondere einen Photomultiplier und/oder eine Photodiode aufweist und/oder dass eine Steuereinrichtung (66) zum automatischen Ansteuern der Elemente der Fluidikeinrichtung (16) und/oder eine Auswerteeinrichtung (58) zum Auswerten der von der Erfassungseinrichtung (54) erfassten Chemolumineszenzphotonen (60) vorgesehen ist.

10. Verfahren zur automatischen Bestimmung von Biomasse (12) in einer Probe (14) mit den Schritten
a) automatische Aufnahme der Probe (14) mit der Biomasse (12) in eine Fluidikeinrichtung (16);
b) automatisches Aktivieren von Chemolumineszenz der Probe (14) in der Fluidikeinrichtung (16);
c) Erfassen von von der Probe (14) emittierten Chemolumineszenzphotonen (60).

11. Verfahren nach Anspruch 10,
**dadurch gekennzeichnet, dass** die Probe (14) vor Schritt b) automatisch wenigstens einmal gefiltert wird, wobei zum Bilden wenigstens eines Filtrats (72, 76) der Probe (14) insbesondere größere Partikel als eine interessierende Biomassespezies und/oder die interessierende Biomassespezies aus der Probe (14) gefiltert werden.

12. Verfahren nach einem der Ansprüche 10 oder 11,
**dadurch gekennzeichnet, dass** in Schritt b) die Probe (14) automatisch mit einem Chemolumineszenzaktivator (20) zur Reaktion gebracht wird, insbesondere einem Chemolumineszenzaktivator (20), der mit ATP und/oder einem Enzym, insbesondere mit alkalischer Phosphatase, reagiert und/oder dass Zellen in der Probe (14) in Schritt b) automatisch lysiert werden.

13. Verfahren nach einem der Ansprüche 10 bis 12,
**dadurch gekennzeichnet, dass** in Schritt c) Chemolumineszenzphotonen (60) in einem ersten Filtrat (76) aus der Probe (14) ohne darin vorhandener interessierender Biomasse (12) und in einem zweiten Filtrat (72) aus der Probe (14) mit darin vorhandener interessierender Biomasse (12) erfasst werden, wobei eine Differenz der erfassten Chemolumineszenzphotonen (60) gebildet wird.

14. Verfahren nach einem der Ansprüche 10 bis 13,
**dadurch gekennzeichnet, dass** nach Schritt c) der Schritt Auswerten der erfassten Chemolumineszenzphotonen (60) durch Vergleich mit zuvor erfassten Datensätzen durchgeführt wird.

15. Verfahren nach einem der Ansprüche 10 bis 14,
**dadurch gekennzeichnet, dass** die Schritte des Verfahrens in einer Detektionsvorrichtung (10) durchgeführt werden, wobei die Elemente der Detektionsvorrichtung (10) derart angesteuert werden, dass die Schritte automatisch erfolgen und/oder dass vor Schritt a) wenigstens teilweise eine Reinigung der Elemente der Detektionsvorrichtung (10) durchgeführt wird.
